# EUROPÄISCHE PATENTSCHRIFT

(11) **EP 1 173 275 B1**
(45) Veröffentlichungstag und Bekanntmachung des Hinweises auf die Patenterteilung: **23.07.2003**
(21) Anmeldenummer: 00929472.9
(22) Anmeldetag: 02.05.2000
(51) Int. Cl.: B01J 19/00

(54) **VERFAHREN UND VORRICHTUNG ZUR PARALLELEN HERSTELLUNG VON OLIGONUKLEOTIDEN**
METHOD AND DEVICE FOR THE PARALLEL MANUFACTURE OF OLIGONUCLEOTIDES
PROCEDE ET DISPOSITIF POUR LA PRODUCTION EN PARALLELE D'OLIGONUCLEOTIDES

(30) Priorität: 29.04.1999 DE 19919607
(43) Veröffentlichungstag der Anmeldung: 23.01.2002
(73) Patentinhaber: Metabion Gesellschaft für Angewandte Biotechnologie mbH, 82152 Planegg-Martinsried (DE)
(72) Erfinder: STÖCKL, Ludwig, D-85586 Poing (DE)
(74) Vertreter: Beyer, Andreas, Dr.
(86) Internationale Anmeldenummer: EP0003916
(87) Internationale Veröffentlichungsnummer: WO01008794

(56) Entgegenhaltungen:
- WO-A-00/18503
- WO-A-00/40330
- WO-A-98/57181
- WO-A-99/65602
- BAIER J ET AL: "SYNTHESIS AND PURIFICATION IN A SINGLE COLUMN ON A HIGH-THROUGHPUT AUTOMATED OLIGONUCLEOTIDE PRODUCTION SYSTEM" BIOTECHNIQUES,US,EATON PUBLISHING, NATICK, Bd. 20, 1996, Seiten 298-303, XP000857679 ISSN: 0736-6205

## Beschreibung

Die vorliegende Erfindung betrifft ein Verfahren und eine Vorrichtung zur parallelen Herstellung von wenigstens 4n Oligonukleotiden.

Aus der DE 42 06 488 A1 sind ein Verfahren und eine Vorrichtung zur Herstellung von Oligonukleotiden bekannt. Die bekannte Vorrichtung weist vier übereinanderliegende Stäbe auf, deren Kontaktflächen durch Schleifen und Polieren so bearbeitet sind, daß die Stäbe spaltfrei relativ zueinander verschoben werden können. Einer der Stäbe enthält Reaktionsräume, die über Zuund Abgangsleitungen in den übrigen Stäben befüllt und entleert werden können. Die einzelnen Reaktionsräume werden nacheinander mit Reagenzien befüllt. Damit die erwähnten Kontaktflächen der verschiebbaren Stäbe gegeneinander gut abdichten, ist eine sehr präzise Bearbeitung dieser Kontaktflächen notwendig und die Stäbe müssen aus verschleißbeständigem Material bestehen, beispielsweise aus rostfreiem Stahl oder aus bestimmten Glaswerkstoffen.

Der Bedarf an Oligonukleotiden steigt ständig und es besteht daher der Wunsch, eine möglichst große Anzahl von Oligonukleotiden kostengünstig, in kurzer Zeit und mit hoher Qualität herzustellen. Dabei kann es sich um gleichartige oder um verschiedene oligonukleotide handeln.

Der Erfindung liegt daher die Aufgabe zugrunde, ein verbessertes Verfahren und eine verbesserte Vorrichtung zur Herstellung von Oligonukleotiden bereitzustellen, die dem zuvor genannten Wunsch Rechnung trägt.

Diese Aufgabe ist erfindungsgemäß durch das im Patentanspruch 1 angegebene Verfahren zur parallelen Herstellung von wenigstens 4n Oligonukleotiden gelöst. Bei dem erfindungsgemäßen Verfahren werden wenigsten vier Einschübe mit je n Reaktionsgefäßen (n ≥ 1 auf bzw. in einem Teller derart angeordnet, daß ein erster Einschub sich an einer ersten Station, ein zweiter Einschub sich an einer zweiten Station, ein dritter Einschub sich an einer dritten Station, und ein vierter Einschub sich an einer vierten Station befinden. Jedes Reaktionsgefäß enthält eine zur Synthese von Oligonukleotiden erforderliche Startbase, die beispielsweise an einen inerten Träger gebunden ist.
Anstelle einer Startbase kann auch ein Fachleuten bekannter, sogenannter Universalträger verwendet werden. Als Trägermaterial kann z.B. poröses Glas, sogenanntes controlled pore glass (CPG) verwendet werden. An den genannten vier Stationen werden dann parallel eine Reihe von Operationen zur Oligonukleotidsynthese durchgeführt.

So wird gleichzeitig in allen n Reaktionsgefäßen des sich an der ersten Station befindenden Einschubs eine sogenannte Deblock-Operation durchgeführt, mit der an den Startbasen vorhandene Schutzgruppen abgespalten werden, um später einzelne Nukleotid-Bausteine an die Startbase ankoppeln zu können. Diese Schutzgruppen werden auch als DMT-Gruppen bezeichnet. Gleichzeitig mit der an der ersten Station stattfindenden Deblock-Operation findet an der zweiten Station, wiederum gleichzeitig in allen n Reaktionsgefäßen, die sich an der zweiten Station befinden, eine erste Wasch-Operation statt, mit der die zuvor abgespaltenen Schutzgruppen aus den Reaktionsgefäßen ausgewaschen werden. Ebenfalls gleichzeitig mit den beiden zuvor genannten Operationen findet in allen sich an der dritten Station befindenden n Reaktionsgefäßen eine sogenannte Coupling-Operation statt, mittels derer die gewünschten einzelnen Nukleotide an die sich in den Reaktionsgefäßen befindenden Startbasen oder Nukleotidketten angekoppelt werden. Wiederum zeitgleich mit den vorgenannten drei Operationen findet gleichzeitig in allen n Reaktionsgefäßen des sich an der vierten Station befindenden Einschubes eine Abfolge von Operationen statt, nämlich zunächst eine zweite Wasch-Operation, an die sich eine sogenannte Capping-Operation anschließt, mittels derer in den Reaktionsgefäßen diejenigen Oligonukleotide blockiert werden, die in der vorangegangenen Coupling-Operation nicht die gewünschte Kettenverlängerung erfahren haben, gefolgt von einer dritten Wasch-Operation, an die sich eine Oxidations-Operation zum Stabilisieren des Phosphat-Grundgerüstes der Oligonukleotide anschließt, und schließlich eine vierte Waschoperation.

Erfindungsgemäß wird entweder der Drehteller stationsweise durch die genannten vier Stationen rotiert, so daß jeder Einschub die einzelnen Stationen nacheinander durchläuft, oder die Stationen werden relativ zu den Einschüben jeweils eine Station weiterbewegt. Diese stationsweise Relativbewegung zwischen den Einschüben und den Stationen erfolgt solange, bis die gewünschten Oligonukleotide durch Aneinanderkoppeln der einzelnen Nukleotide entstanden sind.

Der erfindungsgemäß mittels des Durchlaufens der vier Stationen realisierte Synthesezyklus an sich ist bekannt und braucht daher nicht im einzelnen erläutert zu werden. Allerdings wird mittels des erfindungsgemäßen Verfahrens dieser Synthesezyklus extrem zeitsparend abgearbeitet, indem zum einen alle sich an einer Station befindenden n Reaktionsgefäße zugleich der an dieser Station stattfindenden Operation bzw. den dort stattfindenden Operationen unterzogen werden, und indem zum zweiten parallel an allen vier Stationen Operationen stattfinden. Des weiteren nutzt das erfindungsgemäße Verfahren auf intelligente Weise den Umstand, daß die an der ersten Station ausgeführte Deblock-Operation und die an der dritten Station ausgeführte Coupling-Operation die am längsten dauernden Operationen sind (und damit die notwendige Verweilzeit pro Station vorgeben), indem die deutlich schnelleren capping- und Oxidations-Operationen nacheinander an nur einer Station (der vierten Station) durchgeführt werden.

Beide Maßnahmen führen dazu, daß das erfindungsgemäße Verfahren gegenüber bekannten Verfahren zur Oligonukleotidsynthese erheblich schneller ist. Die Produktivität ist auf diese Weise erhöht und die Herstellkosten für Oligonukleotide können gesenkt werden. Darüber hinaus können größere Mengen an Oligonukleotiden in kürzerer Zeit zur Verfügung gestellt werden. Sind beispielsweise pro Einschub 24 Reaktionsgefäße vorhanden und wird als Taktzeit pro Station eine Zeitdauer von 60 Sekunden veranschlagt, dann lassen sich innerhalb von ca. 90 Minuten 96 Oligonukleotide mit je 20 Nukleotidbausteinen erzeugen. Geht man ferner davon aus, daß etwa alle 100 Minuten ein neuer Durchlauf gestartet werden kann, erreicht man mit dem erfindungsgemäßen Verfahren eine Produktion von 1.350 Oligonukleotiden pro Tag. Dies entspricht nahezu der 40-fachen Menge, die mit dem Gerät gemäß der eingangs genannten DE 42 06 488 A1 hergestellt werden kann.

Gemäß einer bevorzugten Ausgestaltung des erfindungsgemäßen Verfahrens findet an der zweiten Station zusammen mit der dort durchgeführten ersten Wasch-Operation eine Monitoring-Operation statt, die Aufschluß über die Qualität der an der ersten Station durchgeführten Deblock-Operation gibt. Vorzugsweise erfolgt diese Monitoring-Operation mittels einer Online-Messung der Leitfähigkeit einer für die erste Wasch-Operation verwendeten Waschflüssigkeit. Alternativ kann die Monitoring-Operation mittels einer Online-Messung der Farbintensität der für die erste Wasch-Operation verwendeten Waschflüssigkeit erfolgen, wobei insbesondere eine Messung mittels UV-Licht verwendet wird. Die Wellenlänge des verwendeten UV-Lichtes hat vorzugsweise eine Wellenlänge von 455 - 465 nm.

Gemäß einer besonders bevorzugten Ausgestaltung des erfindungsgemäßen Verfahrens wird dann, wenn an der zweiten Station zusätzlich zur ersten Wasch-Operation auch die Monitoring-Operation stattfindet, die erste Wasch-Operation solange durchgeführt, bis die Monitoring-Operation ergibt, daß die in der vorangegangenen Deblock-Operation entfernten Schutzgruppen vollständig ausgespült sind. Der Begriff "vollständig" ist dabei nicht in einem absoluten Sinne zu verstehen, sondern bezieht sich auf die Nachweisgrenze des im Rahmen der Monitoring-Operation verwendeten Meßverfahrens. Durch eine solche Verfahrensweise wird der Verbrauch an Waschflüssigkeit reduziert, weil aufgrund der kontinuierlichen Überwachung und Auswertung der die Reaktionsgefäße verlassenden Waschflüssigkeit die erste Wasch-Operation sofort beendet wird, wenn das gewünschte Ergebnis erreicht ist. Des weiteren wird mit einer solchen Verfahrensweise eine Qualitätssteigerung erzielt, da die erste Wasch-Operation nicht nach einer vorgegebenen Zeitdauer beendet wird, sondern ihre Beendigung vom Erreichen eines bestimmten Ergebnisses abhängt.

Vorteilhaft wird in dem erfindungsgemäßen Verfahren bei der an der dritten Station durchgeführten Coupling-Operation eine ausgewählte Nukleotid-Base gleichzeitig mit einem Aktivator (Katalysator) den Reaktionsgefäßen zugegeben. Diese gleichzeitige Zugabe von Nukleotid-Baustein und Aktivator spart wertvolle Zeit bei einer Operation, die wie oben ausgeführt taktzeitbestimmend ist. Als Aktivator wird vorzugsweise Tetrazol verwendet.

Des weiteren wird bei der an der dritten Station durchgeführten Coupling-Operation falls gewünscht auch eine Markierungsgruppe (label) den Reaktionsgefäßen zugegeben, die eine spätere Identifikation des erzeugten Oligonukleotids oder eines daraus erzeugten Produktes erleichtert. Die Markierungsgruppe ist insbesondere ein Basenanalogon, ein Farbstoff oder ein Hapten.

Das erfindungsgemäße Verfahren ist vorzugsweise als Durchflußverfahren ausgestaltet, d.h. die Reaktionsgefäße sind als Durchflußgefäße ausgebildet, beispielsweise indem jedes Reaktionsgefäß oben und unten durch einen Frittenboden verschlossen ist. Die den Reaktionsgefäßen zuzuführenden Flüssigkeiten (Reagenzien, Waschflüssigkeit etc.) werden bei einem solchermaßen ausgestalteten Verfahren vorzugsweise durch Anlegen eines Druckgefälles zwischen jedem Reaktionsgefäßeinlaß und jedem Reaktionsgefäßauslaß in die einzelnen Reaktionsgefäße hinein und aus ihnen heraus befördert. Das Druckgefälle kann entweder durch einen Überdruck in den Vorratsbehältern für die Reagenzien, Waschflüssigkeiten etc. oder durch Anlegen eines Unterdrucks an die Reaktionsgefäßauslässe erzeugt werden. Dabei muß der Einlaß jedes Reaktionsgefäßes nicht unbedingt oben angeordnet sein, sondern kann sich durchaus auch unten befinden, während der Rektionsgefäßauslaß oben angeordnet ist. Eine solche Anordnung hat den Vorteil, daß die jedem Reaktionsgefäß von unten zugegebenen Flüssigkeiten und Reagenzien das in den Reaktionsgefäßen enthaltene Trägermaterial, beispielsweise die Glaskügelchen, hochwirbeln und auf diese Weise eine bessere Durchmischung der zugegebenen Flüssigkeit im Reaktionsgefäß ermöglichen.

Die eingangs genannte Aufgabe ist erfindungsgemäß auch durch eine Vorrichtung zur parallelen Herstellung von wenigstens 4n Oligonukleotiden gelöst, die eine erste Station zur Durchführung einer Deblock-Operation, eine zweite Station zur Durchführung einer ersten Wasch-Operation, eine dritte Station zur Durchführung einer Coupling-Operation, und eine vierte Station aufweist, an der nacheinander eine zweite Wasch-Operation, eine Capping-Operation, eine dritte Wasch-Operation, eine Oxidations-Operation und eine vierte Wasch-Operation durchgeführt werden. Die genannten vier Stationen sind in Umfangsrichtung aufeinanderfolgend angeordnet und haben voneinander in Umfangsrichtung vorzugsweise einen gleichen Abstand.

Die erfindungsgemäße Vorrichtung umfaßt des weiteren einen Teller, in dem wenigstens vier Einschübe mit je n Reaktionsgefäßen derart angeordnet werden können, daß ein erster Einschub sich an der ersten Station, ein zweiter Einschub sich an der zweiten Station, ein dritter Einschub sich an der dritten Station, und ein vierter Einschub sich an der vierten Station befindet. Vorzugsweise ist der die Einschübe aufnehmende Teller als Drehteller ausgebildet, der stationsweise durch die genannten vier Stationen rotiert werden kann. Ebenso ist es jedoch möglich, die Stationen relativ zu dem die Einschübe enthaltenden Teller zu bewegen.

Jedes Reaktionsgefäß ist als Durchflußgefäß mit einem Reaktionsgefäßeinlaß und einem gegenüber angeordneten Reaktionsgefäßauslaß ausgeführt und vorzugsweise vertikal angeordnet. In jeder der vier Stationen ist den Reaktionsgefäßeinlässen eine Flüssigkeits-Zuführeinrichtung zugeordnet. Die der dritten Station zugeordnete Zuführeinrichtung weist vorzugsweise n Zuführventile auf, damit jedem Reaktionsgefäß selektiv ein bestimmter Nukleotidbaustein zugegeben werden kann. In den übrigen Stationen ist es nicht erforderlich, die Zuführeinrichtung mit n Zuführventilen zu versehen, denn die dort ablaufenden Operationen werden hinsichtlich aller Reaktionsgefäße mit den gleichen Reagenzien oder Flüssigkeiten durchgeführt. Unter bestimmten Umständen ist es allerdings auch für die Zuführeinrichtungen der restlichen Stationen wünschenswert, diese mit jeweils n Zuführventilen zu versehen, beispielsweise dann, wenn unterschiedlich lange Oligonukleotide erzeugt werden sollen. Es ist dann nämlich möglich, die kürzerkettigen, bereits fertigen Oligonukleotide von weiteren, unnötigen Deblock-Operationen auszunehmen, indem die den entsprechenden Reaktionsgefäßen zugeordneten Zuführventile der ersten Station einfach nicht mehr geöffnet werden. Eine wiederholt ausgeführte, unnötige Deblock-Operation kann nämlich bei bereits fertigen Oligonukleotiden zu einer Qualitätsverschlechterung führen, weil einzelne Basen der Oligonukleotide herausgetrennt werden, wodurch das betreffende Oligonukleotid verändert bzw. zerstört wird. Des weiteren läßt sich dann, wenn die Zuführeinrichtung jeder Station mit n Zuführventilen versehen ist, der Verbrauch an Reagenzien und anderen Flüssigkeiten minimieren, weil diese nur noch den Reaktionsgefäßen zugeleitet werden, in denen sie tatsächlich benötigt werden.

Jedem Reaktionsgefäßauslaß ist in jeder Station ein Ablaßkanal zugeordnet, in den die aus den Reaktionsgefäßen austretenden Flüssigkeiten fließen. Die erfindungsgemäße Vorrichtung ist so ausgestaltet, daß sich dann, wenn sich die Einschübe in einer Station befinden, jeder Reaktionsgefäßeinlaß in dichtendem Eingriff mit der zugehörigen Zuführeinrichtung und jeder Reaktionsgefäßauslaß in dichtendem Eingriff mit dem zugehörigen Ablaßkanal befindet. Die Reaktionsgefäßeinlässe sind unten und die Reaktionsgefäßauslässe sind oben angeordnet, so daß den Reaktionsgefäßen die Reagenzien und die sonstigen Flüssigkeiten von unten zugegeben werden. Dies kann bezüglich der Durchmischung in den Reaktionsgefäßen von Vorteil sein.

Wenn eine Relativbewegung um eine Station durchgeführt wird, besteht zumindest zwischen den Reaktionsgefäßeinlässen und der Zuführeinrichtung bzw. den Zuführventilen ein kleiner axialer Abstand. Auf diese Weise ist der Verschleiß der Dichtflächen deutlich herabgesetzt und es besteht dennoch kaum eine Möglichkeit des Zutritts unerwünschter Stoffe zu den Reaktionsgefäßen.

Gemäß einer Ausgestaltung der erfindungsgemäßen Vorrichtung befindet sich letztere in einem abgeschlossenen Raum mit einer Inertgas-Umgebung, so daß die Oligonukleotidsynthese nicht durch Wasser bzw. Wasserdampf beeinträchtigt werden kann. Das Inertgas, beispielsweise Argon oder Stickstoff, kann vorteilhaft oben auf die erfindungsgemäße Vorrichtung kontinuierlich aufgegeben werden und sinkt dann langsam über die Vorrichtung herab. Auf diese Weise ist der Inertgasverbrauch vermindert, denn es wird lediglich die Vorrichtung selbst mit einem schützenden Mantel aus Inertgas umgeben.

Bei einer Ausführungsform der erfindungsgemäßen Vorrichtung besteht auch zwischen den Reaktionsgefäßauslässen und den Ablaßkanälen ein kleiner axialer Abstand, während die stationsweise Relativbewegung stattfindet. Vorzugsweise ist die erfindungsgemäße Vorrichtung dabei so ausgestaltet, daß alle Flüssigkeits-Zuführeinrichtungen in oder auf einer Ventilträgerplatte und alle Ablaßkanäle in einer Absaugplatte aufgenommen sind. Die erfindungsgemäße Vorrichtung umfaßt demnach in dieser Ausführungsform als wesentliche Bestandteile drei übereinander angeordnete Platten, von denen die mittlere Platte der Teller ist. Die Ventilträgerplatte und die Absaugplatte haben eine plane Anlagefläche zum Teller und die Reaktionsgefäßeinlässe sind vorzugsweise bündig mit der Oberseite des Tellers und die Reaktionsgefäßauslässe vorzugsweise bündig mit der Unterseite des Tellers ausgebildet, so daß durch einfaches Aneinanderlegen der Ventilträgerplatte, des Tellers und der Absaugplatte eine dichte Verbindung zwischen den Reaktionsgefäßen und den Zuführventilen sowie den Ablaßkanälen erhalten werden kann. Zur einwandfreien und kostengünstigen Abdichtung können beispielsweise Dichtringe aus einem Elastomermaterial in der planen Anlagefläche der Ventilträgerplatte und der planen Anlagenfläche der Absaugplatte aufgenommen sein, die jeden Reaktionsgefäßeinlaß dicht mit dem entsprechenden Zuführventil und jeden Reaktionsgefäßauslaß dicht mit dem zugehörigen Ablaßkanal verbinden.

In einer besonders bevorzugten Ausgestaltung der erfindungsgemäßen Vorrichtung sind die Ventilträgerplatte und die Absaugplatte drehfest und der Drehteller sowie die Absaugplatte sind relativ zur Ventilträgerplatte absenkbar. Zum Drehen des Drehtellers um eine Station wird letzterer zusammen mit der Absaugplatte geringfügig gegenüber der Ventilträgerplatte abgesenkt, um eine Station gedreht, und dann wieder in Anlage mit der Ventilträgerplatte nach oben verfahren. Dabei wird die Absaugplatte etwas weiter als der Drehteller abgesenkt, so daß zwischen der Absaugplatte und dem Drehteller ein kleiner Spalt besteht und die vorhandenen Dichtungen beim Drehen des Drehtellers keiner Scherbelastung ausgesetzt sind. Beim Hochfahren des Drehtellers im Anschluß an dessen Drehung wird auch die Absaugplatte wieder in dichtende Anlage mit der Unterseite des Drehtellers gefahren.

Bei einer alternativen Ausführungsform der erfindungsgemäßen Vorrichtung weist zumindest die Flüssigkeits-Zuführeinrichtung der dritten Station mehrere Einzelzuführungen auf, von denen jede mit nur einer der an der Station den Reaktionsgefäßen zuzugebenden unterschiedlichen Flüssigkeiten in Flüssigkeitsverbindung steht und deren Anzahl wenigstens der Zahl der an der Station den Reaktionsgefäßen zuzugebenden unterschiedlichen Flüssigkeiten entspricht. Ein Kopplungsantrieb kann jede Einzelzuführung wahlweise mit einem Reaktionsgefäßeinlaß verbinden. Die Einzelzuführungen sind auf einem gemeinsamen Träger angeordnet, der quer zu den Reaktionsgefäßeinlässen verschiebbar ist. Vorzugsweise ist der Träger als positionsweise verschiebbarer Wagen ausgebildet, wobei jeder Reaktionsgefäßeinlaß eine Position darstellt.

Mit Vorteil ist eine solche Flüssigkeits-Zuführeinrichtung so ausgestaltet, daß der Kopplungsantrieb in einer Stellung des Trägers, in der Einzelzuführungen mit Reaktionsgefäßeinlässen fluchten, nur diejenige Einzelzuführung mit einem Reaktionsgefäßeinlaß verbindet, die mit der im betreffenden Reaktionsgefäß gerade benötigten Flüssigkeit in Verbindung steht, und daß dann je nach Erfordernis der Träger gegebenenfalls mehrmals in eine andere Stellung verschoben wird, in der Einzelzuführungen mit Reaktionsgefäßeinlässen fluchten und in der wiederum der Kopplungsantrieb nur diejenige Einzelzuführung mit einem Reaktionsgefäßeinlaß verbindet, die mit der im betreffenden Reaktionsgefäß gerade benötigten Flüssigkeit in Verbindung steht. Auf diese Weise wird eine rationelle Zuführung der in den einzelnen Reaktionsgefäßen benötigten Flüssigkeiten erreicht, ohne daß es zu einer Vermischung oder Verschleppung der verschiedenen Flüssigkeiten kommen kann. Der Qualität der hergestellten Oligonukleotide kommt dies zugute.

Bei allen Ausführungsformen der erfindungsgemäßen Vorrichtung erstrecken sich die Einschübe vorzugsweise radial bezüglich des Drehtellers, wobei die Reaktionsgefäße in zumindest einer Reihe angeordnet sind. Zur Vergrößerung der Kapazität der erfindungsgemäßen Vorrichtung weist jeder Einschub vorzugsweise zwei zueinander parallele, sich radial erstreckende Reihen Reaktionsgefäße auf.

Die Einschübe der erfindungsgemäßen Vorrichtung bestehen vorzugsweise aus Kunststoff, insbesondere aus PEEK (Polyetheretherketon). Es ist bei der erfindungsgemäßen Vorrichtung nicht notwendig, die Einschübe aus Edelstahl oder aus Glaskeramik herzustellen, da die Einschübe keiner abrasiven Beanspruchung unterliegen.

Jeder Einschub ist gemäß einer bevorzugten Ausführungsform so ausgestaltet, daß er auf seinen Längsseiten mindestens eine Codiernut und/oder einen Codiervorsprung aufweist. Die Codiernut eines Einschubs wirkt mit einem komplementär geformten Codiervorsprung des Drehtellers zusammen und ein Codiervorsprung des Einschubs wirkt mit einer komplementär geformten Codiernut des Drehtellers zusammen. Bei jedem Einschub ist mindestens die Codiernut oder der Codiervorsprung verschieden von den anderen Einschüben ausgeführt, so daß ein bestimmter Einschub nur an einer bestimmten Stelle in den Drehteller eingeschoben werden kann. Die Einschübe können auch mehrere Codiernuten und/oder Codiervorsprünge aufweisen.

Vorzugsweise sind die Codiernuten und Codiervorsprünge der Einschübe so ausgestaltet, daß alle Einschübe einer erfindungsgemäßen Vorrichtung zu Reaktionsgefäßplatten zusammensetzbar sind, beispielsweise zu Reaktionsgefäßplatten im MTP-Format. Das weitere Handling der mittels der erfindungsgemäßen Vorrichtung hergestellten Oligonukleotide wird so vereinfacht.

Bei einer vorteilhaften Ausführungsform der erfindungsgemäßen Vorrichtung weist der Reaktionsgefäßauslaß und/oder der Reaktionsgefäßeinlaß jedes Reaktionsgefäßes eine Verengungsstelle auf, die von einer beweglichen Kugel verschließbar ist. Die Kugel wird vorzugsweise durch Schwerkraft, durch Federkraft, oder durch Magnetkraft in Richtung auf die Verengungsstelle gedrängt.

Das erfindungsgemäße Verfahren wird anhand eines Ausführungsbeispiels der erfindungsgemäßen Vorrichtung unter Bezugnahme auf die beigefügten schematischen Figuren näher erläutert. Es zeigt:
- Fig. 1: ein Funktionsschema eines Ausführungsbeispiel einer erfindungsgemäßen Vorrichtung zur parallelen Herstellung von 96 Oligonukleotiden,
- Fig. 2: einen in der erfindungsgemäßen Vorrichtung verwendeten Einschub im in der Vorrichtung befindlichen Zustand,
- Fig. 3: eine Reihe unterschiedlich kodierter Einschübe, von denen jeweils vier zu einer Reaktionsgefäßplatte im MTP-Format kombinierbar sind,
- Fig. 4: eine besondere Verschlußeinrichtung für den Reaktionsgefäßauslaß oder Reaktionsgefäßeinlaß eines Reaktionsgefäßes, und
- Fig. 5 und 6: eine besondere Ausgestaltung einer Flüssigkeits-Zuführeinrichtung der erfindungsgemäßen Vorrichtung.

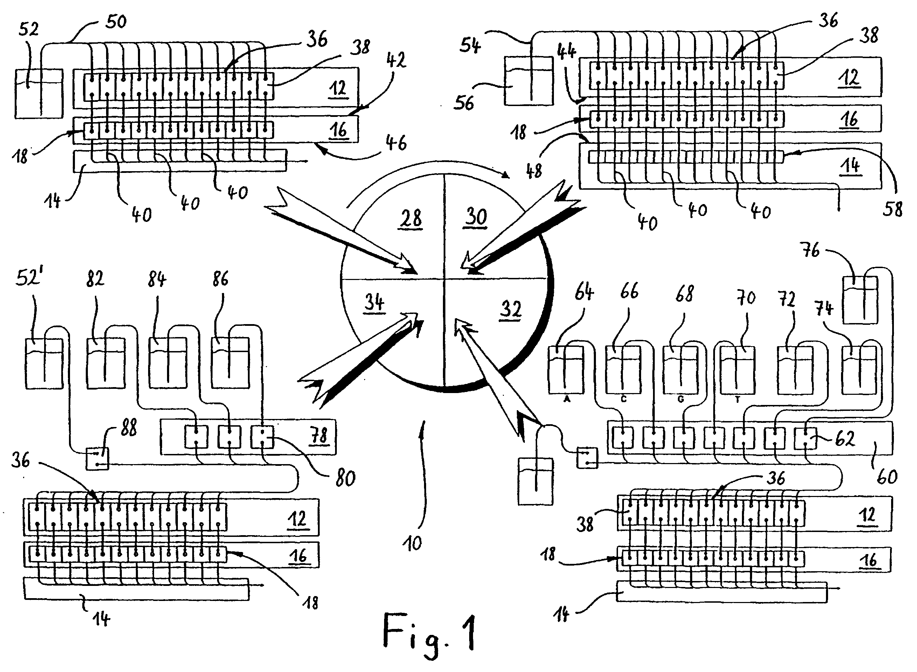

Figur 1 zeigt in der Mitte schematisiert eine Vorrichtung 10 zur parallelen Herstellung von 96 Oligonukleotiden. Wesentliche Bestandteile der Vorrichtung 10 sind drei übereinander angeordnete plattenförmige Gebilde, nämlich eine oben angeordnete Ventilträgerplatte 12, eine unten angeordnete Absaugplatte 14 und ein zwischen diesen beiden Platten angeordneter Drehteller 16. Dieser Drehteller 16 kann relativ zu der drehfesten Ventilträgerplatte 12 und der ebenfalls drehfesten Absaugplatte 14 gedreht werden.

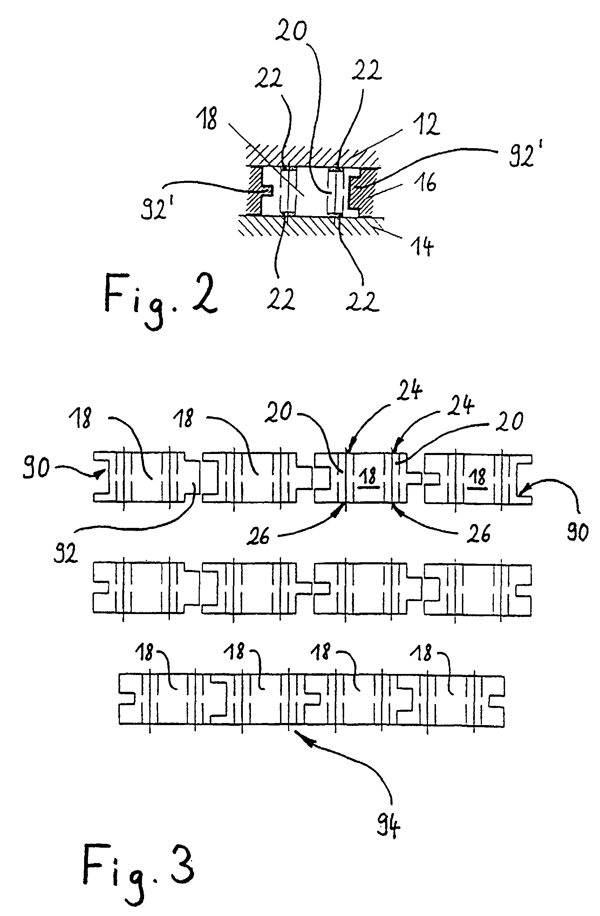

In dem Drehteller 16 befinden sich vier herausnehmbare Einschübe 18, in denen jeweils vierundzwanzig Reaktionsgefäße 20 ausgebildet sind, die in zwei parallelen Reihen zu je zwölf Reaktionsgefäßen 20 angeordnet sind (siehe Figur 2). Jedes Reaktionsgefäß 20 ist oben und unten durch je einen Frittenboden 22 verschlossen und enthält Glaskügelchen aus CPG (controlled pore glass), an die die jeweiligen Startbasen gebunden sind. Jedes Reaktionsgefäß 20 hat oben einen Reaktionsgefäßeinlaß 24 und unten einen Reaktionsgefäßauslaß 26. Die vier Einschübe 18 sind in dem runden Drehteller 16 unter einem Abstand von jeweils 90° zum nächsten Einschub 18 derart angeordnet, daß sie radial in den Drehteller 16 eingeführt und aus ihm herausgezogen werden können.

Die Vorrichtung 10 weist vier separate Stationen 28, 30, 32, 34 auf, an denen bestimmte, zur Oligonukleotidsynthese erforderliche Operationen durchgeführt werden. Die Stationen 28, 30, 32 und 34 folgen in Drehrichtung r des Drehtellers 16 im Abstand von jeweils 90° aufeinander. An jeder Station 28, 30, 32, 34 ist in oder auf der Ventilträgerplatte 12 eine Flüssigkeits-Zuführeinrichtung 36 mit vierundzwanzig Zuführventilen 38 derart angeordnet, daß jedem Reaktionsgefäßeinlaß 24 ein Zuführventil 38 zugeordnet ist. An jeder Station 28, 30, 32, 34 ist des weiteren unterhalb jedes Reaktionsgefäßauslasses 26 ein Ablaßkanal 40 in der Absaugplatte 14 ausgebildet, durch den aus dem zugehörigen Reaktionsgefäß 20 austretende Flüssigkeit abgeführt wird.

Der Drehteller 16 ist mittels einer nicht dargestellten Hubund Senkeinrichtung relativ zur Ventilträgerplatte 12 absenkbar. Mit derselben Hub- und Senkeinrichtung kann auch die Absaugplatte 14 relativ zur Ventilträgerplatte 12 und zum Drehteller abgesenkt und wieder angehoben werden. Mittels der Hub- und Senkeinrichtung können demnach die Reaktionsgefäßeinlässe 24 aller Reaktionsgefäße 20 dichtend an die zugeordneten Zuführventile 38 und die Ablaßkanäle 40 dichtend an die zugeordneten Reaktionsgefäßauslässe 26 angelegt werden. Bei der dargestellten Ausführungsform hat der Drehteller 16 eine plane Oberseite 42, die mit einer planen Anlagefläche 44 der Ventilträgerplatte 12 zusammenwirkt, und eine plane Unterseite 46, die mit einer planen Anlagefläche 48 der Absaugplatte 14 zusammenwirkt. Die eigentliche Abdichtung zwischen der Oberseite 42 des Drehtellers 16 und der Anlagefläche 44 und zwischen der Unterseite 46 des Drehtellers 16 und der Anlagefläche 48 übernehmen O-Ringe aus Elastomermaterial, die um jeden Reaktionsgefäßeinlaß 24 und jeden Reaktionsgefäßauslaß 26 herum angeordnet sind und entweder in die Oberseite 42 oder die Anlagefläche 44 und in die Anlagefläche 48 oder die Unterseite 46 des Drehtellers 16 eingelassen sind.

Im folgenden wird die Funktion der Vorrichtung 10 zur Oligonukleotidsynthese näher erläutert. Angenommen sei dabei, daß sich in jedem Reaktionsgefäß 20 ein Festphasenträger (feinkörniges CPG-Glassubtrat) befindet, an den ein erstes Nukleotid (Startbase) gebunden ist. Weitere Nukleotide sollen an dieses erste Nukleotid gebunden werden. Erwähnt sei, daß jedes neu hinzugegebene Nukleotid sich nur mit einem seiner Enden, dem sogenannten 3'-Ende ankoppeln kann, weil sein anderes Ende, das sogenannte 5'-Ende, mit einer Schutzgruppe (DMT-Gruppe) versehen ist, die verhindert, daß die dem Reaktionsgefäß 20 neu zugegebenen Nukleotide untereinander reagieren.

Beginnend an einer ersten Station 28 werden in einer sogenannten Deblock-Operation die DMT-Gruppen (Schutzgruppen) von dem an dem Festphasenträger gebundenen Startnukleotid (oder einer mittlerweile gebildeten Nukleotidkette) abgespalten, um das 5'-Ende des Nukleotidstrangs reaktionsfähig zu machen. Die Abspaltung der Schutzgruppen wird durch Zugabe von TCA (Trichloressigsäure; genauer 3%-ige Trichloressigsäure in Dichlormethan) erreicht. Hierzu werden die Zuführventile 38 der ersten Station 28, die über eine Leitung 50 mit einem Vorratsbehälter 52 für TCA verbunden sind, 5 Sekunden geöffnet und dann wieder geschlossen. Die den Reaktionsgefäßen 20 zugegebene TCA-Lösung verbleibt dann 50 Sekunden lang in den Reaktionsgefäßen 20. Sodann wird durch Anlegen von Unterdruck an die Ablaßkanäle 40 der ersten Station 28 die TCA-Lösung aus den Reaktionsgefäßen 20 abgesaugt.

Der Drehteller 16 und die Absaugplatte 14 werden dann abgesenkt, bis sich zwischen der Oberseite 42 des Drehtellers 16 und der Anlagefläche 44 sowie zwischen der Unterseite 46 des Drehtellers 16 und der Anlagefläche 48 ein kleiner Spalt gebildet hat. Darauf hin wird der Drehteller 16 um 90° im Uhrzeigersinn gedreht, so daß der sich zu Beginn in der ersten Station 28 befindende Einschub 18 mit seinen Reaktionsgefäßen 20 sich nunmehr in der zweiten Station 30 befindet. Die Absaugplatte 14 und der Drehteller 16 werden wieder in dichtende Anlage miteinander und mit der Ventilträgerplatte 12 gefahren.

In der zweiten Station 30 findet eine erste Wasch-Operation statt, während der die Reaktionsgefäße 20 intensiv gespült werden. Dies geschieht durch Zugabe von Acetonitril, wozu die Zuführventile 38 der zweiten Station 30, die über eine Leitung 54 mit einem Acetonitril-Vorratsbehälter 56 verbunden sind, geöffnet werden. Die Acetonitril-Lösung läuft durch die Reaktionsgefäßeinlässe 24 in jedes Reaktionsgefäß 20 und aus den Reaktionsgefäßauslässen 26 wieder heraus in die Ablaßkanäle 40 der zweiten Station 30.

In die Ablaßkanäle 40 der zweiten Station 30 integriert ist eine Meßeinrichtung 58, mittels derer online die Qualität der an der ersten Station 28 stattgefundenen Deblock-Operation überprüft werden kann. Hierzu wird die aus den Reaktionsgefäßen 20 strömende Acetonitril-Lösung durch Messung ihrer Leitfähigkeit oder durch Messung ihrer Farbintensität untersucht. Die in der Deblock-Operation entfernten Schutzgruppen lassen sich beispielsweise optisch als orange Einfärbung der Acetonitril-Lösung erkennen. Stellt die Meßeinrichtung 58 keine solche Einfärbung mehr fest, kann davon ausgegangen werden, daß alle entfernten Schutzgruppen aus den Reaktionsgefäßen 20 herausgespült worden sind. Veranschlagt wird hierfür eine Zugabezeit von 15 Sekunden, jedoch wird die Acetonitril-Lösung aus dem Behälter 56 den Reaktionsgefäßen 20 mittels der Zuführventile 38 solange zugeführt, bis keine Einfärbung der die Reaktionsgefäße verlassenden Flüssigkeit mehr festgestellt werden kann.

Sodann wird der Drehteller 16 wie zuvor beschrieben eine Station weiter gedreht. Der betrachtete Einschub 18 befindet sich damit in der dritten Station 32. Dort findet die eigentliche, kettenverlängernde Reaktion statt, die als Coupling-Operation bezeichnet wird. Zur Kettenverlängerung muß den sich in den Reaktionsgefäßen 20 befindenden Nukleotidketten eine weitere Nukleotid-Base zugegeben werden, die dann an das in der zweiten Station 30 reaktionsfähig gemachte Ende der Nukleotidkette ankoppelt. Zur Auswahl der gewünschten Nukleotid-Base steht die der dritten Station 32 zugeordnete Flüssigkeits-Zuführeinrichtung 36 in fluidleitender Verbindung mit einem Auswahlventilblock 60, der sieben Auswahlventile 62 aufweist. Je eines der Auswahlventile 62 ist mit einem Adenosin-Vorratsbehälter 64, einem Cytosin-Vorratsbehälter 66, einem Guanosin-Vorratsbehälter 68 und einem Thymidin-Vorratsbehälter 70 verbunden. Ein weiteres Auswahlventil 62 ist mit einem Vorratsbehälter 72 für Tetrazol verbunden, das als Aktivator dient. Die zwei restlichen Auswahlventile 62 sind mit Vorratsbehältern 74 und 76 verbunden, die beispielsweise Markierungsreagenzien enthalten können, etwa oder ein Farbstoff und Hapten. Solche Markierungsreagenzien können während der Coupling-Operation zugegeben werden, um später eine Identifikation eines mit dem Oligonukleotid erzeugten Produktes zu ermöglichen.

Durch Öffnen des der gewünschten Nukleotid-Base zugeordneten Auswahlventiles 62 und der entsprechenden Zuführventile 38 kann die ausgewählte Nukleotid-Base den Reaktionsgefäßen 20 zugegeben werden. Als Zugabezeit werden etwa 2,5 Sekunden veranschlagt. Gleichzeitig mit der Nukleotid-Base und/oder kurz davor und, falls erforderlich, auch danach wird der Aktivator durch Öffnen des entsprechenden Auswahlventils 62 (und der Zuführventile 38) den Reaktionsgefäßen 20 zugegeben. Auf die Zugabe der Nukleotid-Basen und des Aktivators folgt eine Wartezeit von etwa 30 Sekunden, um den neu hinzugegebenen Bausteinen Zeit zu geben, an die bestehende Kette anzukoppeln.

In der dritten Station 32 kann allen Reaktionsgefäßen 20 dieselbe Nukleotid-Base zugegeben werden, beispielsweise Cytosin. Ebenso können jedoch unterschiedlichen Reaktionsgefäßen 20 unterschiedliche Nukleotid-Basen zugegeben werden, so daß in den verschiedenen Reaktionsgefäßen 20 unterschiedliche Oligonukleotidketten erzeugt werden. Dies läßt sich auf einfache Weise durch ein aufeinanderfolgendes Öffnen der den entsprechenden Vorratsbehälter 64 bis 70 freigebenden Auswahlventile 62 erreichen, wobei dann immer nur die denjenigen Reaktionsgefäßen 20 zugeordneten Zuführventile 38 geöffnet sind, in die die entsprechende Nukleotid-Base gelangen soll. Zeitlich stellt diese aufeinanderfolgende Zuführung verschiedener ausgewählter Nukleotid-Basen in unterschiedliche Reaktionsgefäße 20 kein Problem dar, denn die erforderliche Wartezeit von 30 Sekunden ist innerhalb der Taktzeit von 60 Sekunden auch dann noch gewährleistet, wenn alle vier Nukleotid-Basen zugegeben werden.

Nach Verstreichen der Wartezeit wird die sich in den Reaktionsgefäßen 20 befindende Reaktionslösung durch die der dritten Station 32 zugeordneten Ablaßkanäle 40 abgesaugt. Sodann wird der Drehteller 16 in der bereits beschriebenen Weise um eine Station weitergedreht, so daß der betrachtete Einschub 18 sich jetzt in der vierten Station 34 befindet.

In der vierten Station 34 finden nacheinander mehrere Operationen statt, nämlich eine zweite Wasch-Operation, eine capping-Operation, eine dritte Wasch-Operation, eine Oxidations-Operation, und eine vierte Wasch-Operation. Hierzu steht die der vierten Station 34 zugeordnete Flüssigkeits-Zuführeinrichtung 36 in flüssigkeitsleitender Verbindung mit einem zweiten Auswahlventilblock 78 mit drei Auswahlventilen 80. Das erste dieser Auswahlventile 80 ist mit einem Vorratsbehälter 82 für ein erstes Capping-Reagens verbunden, das zweite Auswahlventil 80 mit einem Vorratsbehälter 84 für ein zweites Capping-Reagens, und das dritte Auswahlventil 80 mit einem Vorratsbehälter 86 für eine Jodlösung. Des weiteren kann ein Waschflüssigkeitsvorratsbehälter 52', der mit dem Vorratsbehälter 52 identisch sein kann, durch ein Ventil 88 flüssigkeitsleitend mit der Flüssigkeits-Zuführeinrichtung 36 der vierten Station 34 verbunden werden. Durch Öffnen dieses Ventils 88 und aller Zuführventile 38 für etwa 15 Sekunden wird allen Reaktionsgefäßen 20 zur Ausführung der zweiten Wasch-Operation Acetonitril-Lösung zugeführt. Die Acetonitril-Lösung läuft durch die Reaktionsgefäße 20 hindurch und in die Ablaßkanäle 40 der vierten Station 34 hinein.

Dann wird durch Schließen des Ventils 88 und Öffnen der entsprechenden Auswahlventile 80 etwa 3 Sekunden lang das im Vorratsbehälter 82 enthaltene, erste Capping-Reagens und/oder das im Vorratsbehälter 84 enthaltene zweite Capping-Reagens den Reaktionsgefäßen 20 zugeführt. Die mit diesen Reagenzien durchgeführte, sogenannte Capping-Operation ist notwendig, weil nie 100% der in den Reaktionsgefäßen 20 enthaltenen Oligonukleotidketten in der vorhergehenden Coupling-Operation mit den neu hinzugegebenen Nukleotid-Bausteinen reagiert haben. Diejenigen Kettenenden, die in der Coupling-Operation nicht wie vorgesehen reagiert haben, müssen dauerhaft blockiert werden, um die Bildung fehlerhafter Oligonukleotidketten zu vermeiden. Eine Wartezeit ist nach der Zugabe der Capping-Reagenzien nicht zu beachten, da diese Reagenzien äußerst schnell reagieren.

Auf die Capping-Operation folgt eine analog der zweiten Wasch-Operation durchgeführte dritte Wasch-Operation, wiederum für eine Zeitdauer von etwa 15 Sekunden.

Im Anschluß an die dritte Wasch-Operation folgt durch Schließen des Ventils 88 und Öffnen des dem Vorratsbehälter 86 für Jodlösung zugeordneten Auswahlventils 80 eine Oxidations-Operation, indem die Jodlösung aus dem Vorratsbehälter 86 durch die geöffneten Zuführventile 38 den Reaktionsgefäßen 20 etwa 2 Sekunden lang zugeführt wird. Diese Oxidations-Operation stabilisiert durch Aufoxidation des Phosphors von P(III) zu P(V) das Phosphat-Grundgerüst der Oligonukleotidketten.

Schlußendlich wird analog der zweiten und der dritten Wasch-Operation eine vierte Wasch-Operation für 15 Sekunden durchgeführt, um die Jodlösung aus den Reaktionsgefäßen 20 herauszuspülen. Damit ist ein Durchlauf des betrachteten Einschubs 18 beendet, an den sich weitere Durchläufe anschließen können, um die gewünschten Nukleotid-Ketten zu erzeugen. Nachdem die erforderliche Anzahl von Umläufen pro Einschub 18 stattgefunden hat, kann der entsprechende Einschub aus dem Drehteller 16 seitlich entnommen werden und durch einen neuen, Start-Basen enthaltenden Einschub 18 ersetzt werden.

In den Figuren 2 und 3 sind die Einschübe 18 näher erläutert. Jeder Einschub 18 ist im wesentlichen stabförmig und weist zwei zueinander parallele Reihen Reaktionsgefäße 20 auf, beispielsweise 12 Reaktionsgefäße pro Reihe. An seinen Längsseiten ist jeder Einschub 18 mit zumindest einer Codiernut 90 oder mit zumindest einem Codiervorsprung 92 versehen. Durch diese Codiernuten 90 und/oder Codiervorsprünge 92 ist sichergestellt, daß ein bestimmter Einschub 18 nur an einer bestimmten Position in den Drehteller 16 eingeschoben werden kann, nämlich an derjenigen Position, an der der Drehteller 16 Codiernuten 90' (nicht dargestellt) oder Codiervorsprünge 92' aufweist, die den Codiernuten 90 oder den Codiervorsprüngen 92 des jeweiligen Einschubes 18 entsprechen. Eine Fehlbestückung des Drehtellers 16 ist somit nahezu ausgeschlossen.

Als weiterer Vorteil lassen sich die solchermaßen ausgestalteten Einschübe 18 zu Reaktionsgefäßplatten 94 im Standard MTP-Format zusammensetzen (siehe Figur 3), wobei auch dies nur in einer bestimmten, durch die Codiernuten 90 und Codiervorsprünge 92 der entsprechenden Einschübe 18 vorgegebenen Reihenfolge möglich ist.

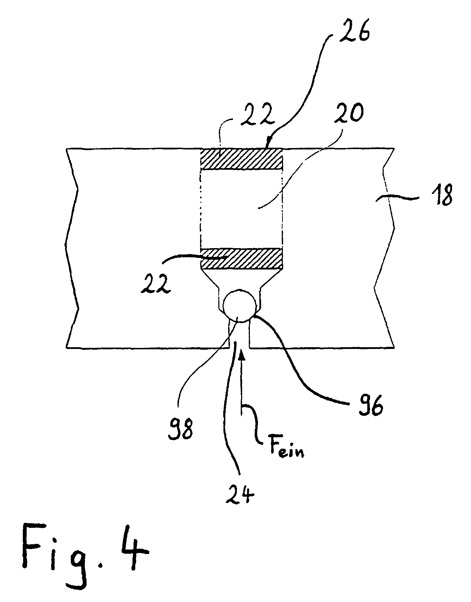

Figur 4 zeigt im Querschnitt ein in einem Einschub 18 ausgebildetes Reaktionsgefäß 20 mit einem unten angeordneten Reaktionsgefäßeinlaß 24 und einem oben angeordneten Reaktionsgefäßauslaß 26 sowie zwei zwischen dem Einlaß 24 und dem Auslaß 26 angeordneten Frittenböden 22. Die Frittenböden 22 begrenzen die eigentliche Reaktionskammer, indem sie durchlässig für Reaktionsflüssigkeiten sind, die Oligonukleotide jedoch nicht aus der Reaktionskammer entweichen lassen.

Während einer Relativbewegung zwischen den Reaktionsgefäßen 20 und den vier Stationen 28, 30, 32 und 34 der Vorrichtung 10 sind die Reaktionsgefäße 20 vom Zu- und Abfluß entkoppelt, d.h. es besteht keine Flüssigkeitsverbindung zwischen einer Zuführleitung und dem Reaktionsgefäßeinlaß 24 und zwischen einer Abführleitung und dem Reaktionsgefäßauslaß 26. Die in dem Reaktionsgefäß 20 befindliche Flüssigkeit kann daher aufgrund der auf sie wirkenden Schwerkraft zum Auslaufen neigen.

Um dies zu verhindern, ist jedes Reaktionsgefäß 20 gemäß einer Ausführungsform der Vorrichtung 10 mit einer speziellen Verschlußeinrichtung 95 für den hier unten liegenden Reaktionsgefäßeinlaß 24 versehen. Diese Verschlußeinrichtung 95 umfaßt eine im Reaktionsgefäßeinlaß 24 angeordnete Verengungsstelle 96, die von einer hin und her beweglichen Kugel 98 verschließbar ist, welche ebenfalls im Reaktionsgefäßeinlaß 24 angeordnet ist.

Im in Figur 4 dargestellten Ausführungsbeispiel ist die Kugel 98 durch Schwerkraft gegen die Verengungsstelle 96 gedrängt. Alternativ oder zusätzlich kann die Kugel 98 aber auch durch eine Feder oder durch magnetische Kräfte in Richtung auf die Verengungsstelle 96 gedrängt sein. Es versteht sich, daß die die Kugel 98 in Richtung auf die Verengungsstelle 96 drängende Kraft kleiner sein muß als die beim Zufließen von Flüssigkeit durch den Reaktionsgefäßeinlaß 24 in Richtung des Pfeils Fₑᵢₙ wirkende Kraft, damit der Reaktionsgefäßeinlaß 24 sich öffnet.

Die Verschlußeinrichtung 95 verhindert nach dem Abkoppeln einer Zuführleitung vom Reaktionsgefäßeinlaß 24 zuverlässig ein Auslaufen von Flüssigkeit aus dem Reaktionsgefäß 20. Obwohl in Figur 4 nur eine dem Reaktionsgefäßeinlaß 24 zugeordnete Verschlußeinrichtung 95 dargestellt ist, ist bei anderen Ausführungsformen eine solche Verschlußeinrichtung 95 auch am Reaktionsgefäßauslaß 26 vorhanden, um bei entkoppeltem Reaktionsgefäß 20 zu verhindern, daß beispielsweise Luft in das Reaktionsgefäß 20 eindringt.

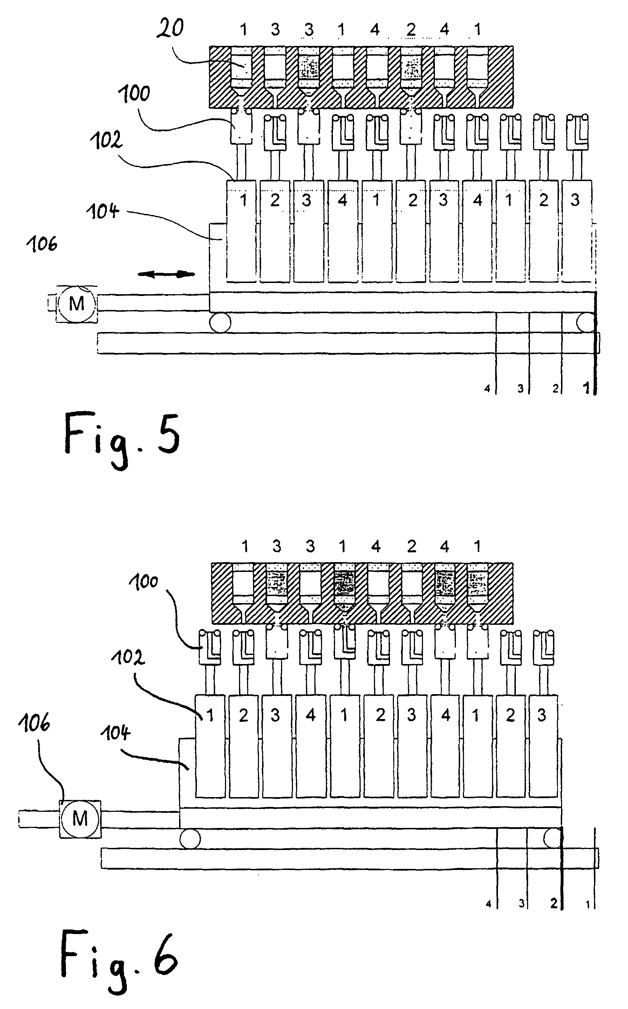

In den Figuren 5 und 6 ist eine spezielle Flüssigkeits-Zuführeinrichtung 36 dargestellt, wie sie insbesondere an der dritten Station 32 der Vorrichtung 10 mit Vorteil Verwendung findet. Diese Flüssigkeits-Zuführeinrichtung 36 weist im dargestellten Ausführungsbeispiel elf Einzelzuführungen 100 auf, von denen jede mit nur einer der an der Station den Reaktionsgefäßen 20 zuzugebenden Flüssigkeiten in Flüssigkeitsverbindung steht. Die Mindestanzahl an Einzelzuführungen 100 entspricht der Zahl unterschiedlicher Flüssigkeiten, die an der jeweiligen Station zuzugeben sind. Vorzugsweise sind jedoch mehr Einzelzuführungen 100 als diese Mindestanzahl vorhanden, damit den in einer Station befindlichen Reaktionsgefäßen 20 die entsprechende Flüssigkeit schneller zugeführt werden kann.

Jede Einzelzuführung 100 ist auf einem Kopplungsantrieb 102 montiert, der die Einzelzuführung 100 dann, wenn sie sich unter dem richtigen Reaktionsgefäßeinlaß 24 befindet, mit diesem Reaktionsgefäßeinlaß 24 koppeln kann. Im dargestellten Ausführungsbeispiel kann der Kopplungsantrieb 102 die ihm zugeordnete Einzelzuführung 100 ein Stück nach oben und nach unten bewegen, um die Kopplung mit und die Entkopplung von dem Reaktionsgefäß 20 zu bewirken. Die Kopplungsantriebe 102 sind zusammen mit den Einzelzuführungen 100 auf einem gemeinsamen Träger 104 befestigt, der quer zu den Reaktionsgefäßeinlässen 24 verschiebbar ist. Im dargestellten Ausführungsbeispiel ist der gemeinsame Träger 104 ein positionsweise verschiebbarer Wagen, derart, daß eine Verschiebung um eine Position dem Abstand zwischen zwei benachbarten Reaktionsgefäßeinlässen 24 entspricht.

Es wird nun die Funktion der in Figuren 5 und 6 dargestellten Flüssigkeits-Zuführeinrichtung 36 erklärt. Zum besseren Verständnis ist über jedem Reaktionsgefäß 20 eine Zahl angegeben, die eine dem entsprechenden Reaktionsgefäß zuzugebende Flüssigkeit symbolisiert. In gleicher Weise ist auf jedem Kopplungsantrieb 102 eine Zahl angegeben, die die Flüssigkeit symbolisiert, mit der die entsprechende Einzelzuführung 100 in Flüssigkeitsverbindung steht. Je nach Stellung des hier als Wagen ausgebildeten Trägers 104 werden von den Kopplungsantrieben 102 nun nur diejenigen Einzelzuführungen 100 mit Reaktionsgefäßen 20 gekoppelt, bei denen die auf dem Kopplungsantrieb angegebene Zahl mit der oberhalb des Reaktionsgefäßes 20 angegebenen Zahl übereinstimmt. Mit anderen Worten, es werden nur diejenigen Einzelzuführungen 100 angekoppelt, die mit der Flüssigkeit in Fluidverbindung stehen, die für das entsprechende Reaktionsgefäß 20 gerade benötigt wird. In Figur 5 sind das von links gesehen die erste, die dritte und die sechste Einzelzuführung 100. Die übrigen Einzelzuführungen 100 bleiben in entkoppelter Stellung.

Nachdem die Flüssigkeiten zugeführt worden sind, entkoppelt der zugehörige Kopplungsantrieb 102 jede Einzelzuführung 100 wieder von dem Reaktionsgefäßeinlaß 24. Sodann fährt der als Wagen ausgebildete Träger 104, angetrieben durch einen Positionsantrieb 106, beispielsweise um eine Position nach links (siehe Fig. 6), so daß die Einzelzuführungen 100 jetzt mit anderen Reaktionsgefäßen 20, genauer deren Reaktionsgefäßeinlässen 24, als vorher fluchten. Nun werden wieder diejenigen Einzelzuführungen 100 mit den Reaktionsgefäßeinlässen 24 gekoppelt, bei denen die Flüssigkeit mit der dem entsprechenden Reaktionsgefäß 20 zuzugebenden Flüssigkeit übereinstimmt. In Figur 6 sind dies die dritte, die fünfte, die achte und die neunte Einzelzuführung 100.

Der beschriebene Vorgang, d.h. das positionsweise Verfahren des Trägers 104, wird solange ausgeführt, bis jedem Reaktionsgefäß 20 die gewünschte Flüssigkeit zugegeben worden ist. Gegebenenfalls kann der Wagen 104 von der Steuerung, mit der er zusammenarbeitet, auch gleich um mehrere Positionen verschoben werden, etwa dann, wenn sich nach Verschieben des Wagens 104 um nur eine Position keine Übereinstimmung zwischen den Flüssigkeiten der Einzelzuführungen 100 und den gerade damit fluchtenden Reaktionsgefäßen 20 ergibt.

Eine solche Flüssigkeits-Zuführeinrichtung 36 ermöglich es, die jeweils benötigten Flüssigkeiten den Reaktionsgefäßen 20 zuzuführen, ohne daß es zu einer Vermischung oder Verschleppung der verschiedenen Flüssigkeiten kommt. Die Qualität der erzeugten Oligonukleotide wird dadurch erhöht.

## Patentansprüche

1. Verfahren zur parallelen Synthese von wenigstens 4n Oligonukleotiden, mit den Schritten:
- Anordnen von wenigstens vier Einschüben mit je n Reaktionsgefäßen auf einem Teller derart, daß ein erster Einschub sich an einer ersten Station, ein zweiter Einschub sich an einer zweiten Station, ein dritter Einschub sich an einer dritten Station und ein vierter Einschub sich an einer vierten Station befinden, wobei jedes Reaktionsgefäß eine an einen inerten Träger gebundene Startbase oder einen Universalträger enthält,
- paralleles Durchführen von
a) einer Deblock-Operation zur Entfernung von Schutzgruppen gleichzeitig in allen n Reaktionsgefäßen des sich an der ersten Station befindenden Einschubes,
b) einer ersten Wasch-Operation gleichzeitig in allen n Reaktionsgefäßen des sich an der zweiten Station befindenden Einschubes,
c) einer Coupling-Operation zum Anfügen einzelner Nukleotide in allen n Reaktionsgefäßen des sich an der dritten Station befindenden Einschubes, und
d) einer zweiten Wasch-Operation gefolgt von einer Capping-Operation zum Blockieren von Oligonukleotiden, die in der vorangegangenen Coupling-Operation nicht die gewünschte Kettenverlängerung erfahren haben, gefolgt von einer dritten Wasch-Operation gefolgt von einer Oxidations-Operation zum Stabilisieren des Phosphatgrundgerüstes der Oligonukleotide, gefolgt von einer vierten Wasch-Operation gleichzeitig in allen n Reaktionsgefäßen des sich an der vierten Station befindenden Einschubs, und
- stationsweises Rotieren der Einschübe durch die genannten vier Stationen oder der Stationen relativ zu den Einschüben und Ausführen der zuvor genannten Operationen solange, bis die gewünschten Oligonukleotide durch Aneinanderkoppeln der einzelnen Nukleotide entstanden sind.

2. Verfahren nach Anspruch 1,
**dadurch gekennzeichnet, daß** an der zweiten Station zusammen mit der dort durchgeführten ersten Wasch-Operation eine Monitoring-Operation stattfindet, die Aufschluß über die Qualität der an der ersten Station durchgeführten Deblock-Operation gibt.

3. Verfahren nach Anspruch 2,
**dadurch gekennzeichnet, daß** die Monitoring-Operation eine Online-Messung der Leitfähigkeit einer für die erste Wasch-Operation verwendeten Waschflüssigkeit umfaßt.

4. Verfahren nach Anspruch 2,
**dadurch gekennzeichnet, daß** die Monitoring-Operation eine Online-Messung der Farbintensität, insbesondere durch Messen mittels UV-Licht, einer für die erste Wasch-Operation verwendeten Waschflüssigkeit umfaßt.

5. Verfahren nach einem der Ansprüche 2 bis 4,
**dadurch gekennzeichnet, daß** die erste Wasch-Operation solange durchgeführt wird, bis die Monitoring-Operation ergibt, daß die in der vorangegangenen Deblock-Operation entfernten Schutzgruppen vollständig ausgespült sind.

6. Verfahren nach einem der vorhergehenden Ansprüche,
**dadurch gekennzeichnet, daß** bei der an der dritten Station durchgeführten Coupling-Operation eine ausgewählte Nukleotid-Base gleichzeitig mit einem Aktivator, vorzugsweise Tetrazol, den Reaktionsgefäßen zugegeben wird.

7. Verfahren nach einem der vorhergehenden Ansprüche,
**dadurch gekennzeichnet, daß** bei der an der dritten Station durchgeführten Coupling-Operation eine Markierungsgruppe, insbesondere ein Basenanalogon, ein Farbstoff oder ein Hapten, den Reaktionsgefäßen zugegeben wird.

8. Verfahren nach einem der vorhergehenden Ansprüche,
**dadurch gekennzeichnet, daß** die Reaktionsgefäße als Durchflußgefäße ausgebildet sind, und daß die in den vier Stationen den Reaktionsgefäßen zuzuführenden Flüssigkeiten durch Anlegen eines Druckgefälles zwischen Reaktionsgefäßeinlaß und Reaktionsgefäßauslaß in die Reaktionsgefäße hinein und aus ihnen heraus befördert werden.

9. Verfahren nach einem der vorhergehenden Ansprüche,
**dadurch gekennzeichnet, daß** alle Operationen unter einer Schutzgasatmospäre durchgeführt werden, insbesondere unter Stickstoff und/oder Argon.

10. Vorrichtung zur parallelen Synthese von wenigstens 4n Oligonukleotiden, mit
- einer ersten Station (28) zur Durchführung einer Deblockoperation,
- einer zweiten Station (30) zur Durchführung einer ersten Wasch-Operation,
- einer dritten Station (32) zur Durchführung einer Coupling-Operation,
- einer vierten Station (34) zur Durchführung einer zweiten Wasch-Operation gefolgt von einer Capping-Operation gefolgt von einer dritten Wasch-Operation gefolgt von einer Oxidations-Operation gefolgt von einer vierten Wasch-Operation, wobei die erste, die zweite, die dritte und die vierte Station in Umfangsrichtung mit Abstand aufeinanderfolgen,
- einem Teller (16), der wenigstens vier Einschübe (18) mit je n Reaktionsgefäßen (20) derart aufweist, daß ein erster Einschub (18) sich an der ersten Station (28), ein zweiter Einschub (18) sich an der zweiten Station (30), ein dritter Einschub (18) sich an der dritten Station (32) und ein vierter Einschub (18) sich an der vierten Station (34) befindet, wobei jedes Reaktionsgefäß (20) als Durchflußgefäß mit einem unten angeordneten Reaktionsgefäßeinlaß und einem oben angeordneten Reaktionsgefäßauslaß ausgebildet ist,
- einer Einrichtung zum Ausführen einer stationsweisen Relativbewegung zwischen dem Teller (16) und den Stationen (28, 30, 32, 34),
- einer in jeder Station (28, 30, 32, 34) unmittelbar den Reaktionsgefäßeinlässen zugeordneten Flüssigkeits-Zuführeinrichtung (36), und
- einem in jeder Station (28, 30, 32, 34) unmittelbar jedem Reaktionsgefäßauslaß zugeordneten Ablaßkanal (40), wobei jeder Reaktionsgefäßeinlaß in dichtendem Eingriff mit der zugehörigen Flüssigkeits-Zuführeinrichtung (36) und jeder Reaktionsgefäßauslaß in dichtendem Eingriff mit dem zugehörigen Ablaßkanal (40) ist, wenn sich die Einschübe (18) in einer Station (28, 30, 32, 34) befinden, und wobei zumindest zwischen den Reaktionsgefäßeinlässen und der Flüssigkeits-Zuführeinrichtung (36) ein kleiner axialer Abstand besteht, während eine stationsweise Relativbewegung zwischen dem Teller (16) und den Stationen (28, 30, 32, 34) stattfindet.

11. Vorrichtung nach Anspruch 10,
**dadurch gekennzeichnet, daß** auch zwischen den Reaktionsgefäßauslässen und den Ablaßkanälen (40) ein kleiner axialer Abstand besteht, während eine stationsweise Relativbewegung zwischen dem Teller (16) und den Stationen (28, 30, 32, 34) stattfindet.

12. Vorrichtung nach Anspruch 10 oder 11,
**dadurch gekennzeichnet, daß** der Teller (16) ein Drehteller ist und daß alle Flüssigkeits-Zuführeinrichtungen (36) in oder auf einer Ventilträgerplatte (12) und alle Ablaßkanäle (40) in einer Absaugplatte (14) aufgenommen sind.

13. Vorrichtung nach einem der Ansprüche 10 bis 12,
**dadurch gekennzeichnet, daß** jede Flüssigkeits-Zuführeinrichtung (36) n Zuführventile (38) aufweist.

14. Vorrichtung nach Anspruch 12 oder 13,
**dadurch gekennzeichnet, daß** die Ventilträgerplatte (12) und die Absaugplatte (14) drehfest sind, und daß der Drehteller (16) und die Absaugplatte (14) relativ zur Ventilträgerplatte (12) absenkbar sind.

15. Vorrichtung nach einem der Ansprüche 10 bis 14,
**dadurch gekennzeichnet, daß** jeder Einschub (18) sich radial im Teller (16) erstreckt und wenigstens eine Reihe, vorzugsweise zwei zueinander parallele Reihen, Reaktionsgefäße (20) aufweist.

16. Vorrichtung nach einem der Ansprüche 10 bis 15,
**dadurch gekennzeichnet, daß** die Einschübe (18) aus Kunststoff bestehen, insbesondere aus Polyetheretherketon.

17. Vorrichtung nach einem der Ansprüche 10 bis 16,
**dadurch gekennzeichnet, daß** jeder Einschub (18) an seinen Längsseiten eine verschieden ausgestaltete Codiernut (90) oder einen Codiervorsprung (92) aufweist, die mit einem entsprechenden Codiervorsprung (90') oder einer entsprechenden Codiernut (92') des Drehtellers (16) zusammenwirken.

18. Vorrichtung nach Anspruch 17,
**dadurch gekennzeichnet, daß** die Einschübe (18) mittels der Codiernuten (90) und Codiervorsprünge (92) zu Reaktionsgefäßplatten (94) zusammensetzbar sind.

19. Vorrichtung nach einem der Ansprüche 10 bis 18,
**dadurch gekennzeichnet, daß** der Reaktionsgefäßauslaß (26) und/oder der Reaktionsgefäßeinlaß (24) jedes Reaktionsgefäßes (20) eine Verengungsstelle (96) aufweist, die von einer beweglichen Kugel (98) verschließbar ist.

20. Vorrichtung nach Anspruch 19,
**dadurch gekennzeichnet, daß** die Kugel (98) durch Schwerkraft, durch Federkraft, oder durch Magnetkraft in Richtung auf die Verengungsstelle (96) gedrängt wird.

21. Vorrichtung nach einem der Ansprüche 10 bis 12,
**dadurch gekennzeichnet, daß** zumindest die Flüssigkeits-Zuführeinrichtung (36) der dritten Station (32) aufweist
- mehrere Einzelzuführungen (100), von denen jede mit nur einer der an der Station den Reaktionsgefäßen (20) zuzugebenden unterschiedlichen Flüssigkeiten in Flüssigkeitsverbindung steht und deren Anzahl wenigstens der Zahl der an der Station den Reaktionsgefäßen (20) zuzugebenden unterschiedlichen Flüssigkeiten entspricht,
- einen Kopplungsantrieb (102) für die Einzelzuführungen (100), um jede Einzelzuführung (100) wahlweise mit einem Reaktionsgefäßeinlaß (24) zu verbinden, und
- einen gemeinsamen Träger (104) für die Einzelzuführungen (100), der quer zu den Reaktionsgefäßeinlässen (24) verschiebbar ist.

22. Vorrichtung nach Anspruch 21,
**dadurch gekennzeichnet, daß** der Träger (104) als positionsweise verschiebbarer Wagen ausgebildet ist, wobei jeder Reaktionsgefäßeinlaß (24) eine Position darstellt.

23. Vorrichtung nach Anspruch 21 oder 22,
**dadurch gekennzeichnet, daß** der Kopplungsantrieb (102) in einer Stellung des Trägers (104), in der Einzelzuführungen (100) mit Reaktionsgefäßeinlässen (24) fluchten, nur diejenige Einzelzuführung (100) mit einem Reaktionsgefäßeinlaß (24) verbindet, die mit der im betreffenden Reaktionsgefäß (20) gerade benötigten Flüssigkeit in Verbindung steht, und daß dann je nach Erfordernis der Träger (104) gegebenenfalls mehrmals in eine andere Stellung verschoben wird, in der Einzelzuführungen (100) mit Reaktionsgefäßeinlässen (24) fluchten und in der wiederum der Kopplungsantrieb (102) nur diejenige Einzelzuführung (100) mit einem Reaktionsgefäßeinlaß (24) verbindet, die mit der im betreffenden Reaktionsgefäß (20) gerade benötigten Flüssigkeit in Verbindung steht.

## Claims

1. Process for the parallel synthesis of at least 4n oligonucleotides, with the steps:
- arrangement of at least four inserts each with n reaction vessels on a plate such that a first insert is at a first station, a second insert is at a second station, a third insert is at a third station and a fourth insert is at a fourth station, each reaction vessel containing an initiator base bound to an inert carrier, or a universal carrier,
- carrying out in parallel of
a) a deblocking operation to remove protective groups simultaneously in all n reaction vessels of the insert at the first station,
b) a first washing operation simultaneously in all n reaction vessels of the insert at the second station,
c) a coupling operation for attaching individual nucleotides in all n reaction vessels of the insert at the third station, and
d) a second washing operation followed by a capping operation for blocking oligonucleotides which have not undergone the desired chain lengthening in the preceding coupling operation followed by a third washing operation followed by an oxidation operation for stabilizing the phosphate foundation matrix of the oligonucleotides, followed by a fourth washing operation simultaneously in all n reaction vessels of the insert at the fourth station, and
- station by station rotation of the inserts through the four stations mentioned or of the stations relative to the inserts and carrying out of the abovementioned operations until the desired oligonucleotides have been formed by coupling of the individual nucleotides to one another.

2. Process according to claim 1,
**characterized in that** at the second station, together with the first washing operation carried out there, a monitoring operation which provides information on the quality of the deblocking operation carried out at the first station takes place.

3. Process according to claim 2,
**characterized in that** the monitoring operation comprises an on-line measurement of the conductivity of a washing liquid used for the first washing operation.

4. Process according to claim 2,
**characterized in that** the monitoring operation comprises an on-line measurement of the colour intensity, in particular by measurement by means of UV light, of a washing liquid used for the first washing operation.

5. Process according to one of claims 2 to 4,
**characterized in that** the first washing operation is carried out until the monitoring operation shows that the protective groups removed in the preceding deblocking operation have been rinsed out completely.

6. Process according to one of the preceding claims,
**characterized in that** in the coupling operation carried out at the third station, a selected nucleotide base is added to the reaction vessels simultaneously with an activator, preferably tetrazole.

7. Process according to one of the preceding claims,
**characterized in that** in the coupling operation carried out at the third station, a marking group, in particular a base analogue, a dyestuff or a hapten, is added to the reaction vessels.

8. Process according to one of the preceding claims,
**characterized in that** the reaction vessels are constructed as flow-through vessels, and **in that** the liquids to be fed to the reaction vessels in the four stations are conveyed into the reactions vessels and out of them by applying a pressure gradient between the reaction vessel inlet and reaction vessel outlet.

9. Process according to one of the preceding claims,
**characterized in that** all the operations are carried out under an inert gas atmosphere, in particular under nitrogen and/or argon.

10. Device for the parallel synthesis of at least 4n oligonucleotides, comprising
- a first station (28) for carrying out a deblocking operation,
- a second station (30) for carrying out a first washing operation,
- a third station (32) for carrying out a coupling operation,
- a fourth station (34) for carrying out a second washing operation followed by a capping operation followed by a third washing operation followed by an oxidation operation followed by a fourth washing operation, the first, the second, the third and the fourth station following one another at a distance in the circumferential direction,
- a plate (16), which has at least four inserts (18) each with n reaction vessels (20) such that a first insert (18) is at the first station (28), a second insert (18) is at the second station (30), a third insert (18) is at the third station (32) and a fourth insert (18) is at the fourth station (34), each reaction vessel (20) being constructed as a flow-through vessel with a reaction vessel inlet arranged at the bottom and a reaction vessel outlet arranged at the top,
- a device for carrying out a relative movement station by station between the plate (16) and the stations (28, 30, 32, 34),
- a liquid feed device (36) assigned directly to the reaction vessel inlets in each station (28, 30, 32, 34), and
- a drain channel (40) assigned directly to each reaction vessel outlet in each station (28, 30, 32, 34), each reaction vessel inlet being in tight engagement with the associated liquid feed device (36) and each reaction vessel outlet being in tight engagement with the associated drain channel (40) when the inserts (18) are in a station (28, 30, 32, 34), and there being a small axial distance at least between the reaction vessel inlets and the liquid feed device (36) while a relative movement station by station between the plate (16) and the stations (28, 30, 32, 34) takes place.

11. Device according to claim 10,
**characterized in that** there is also a small axial distance between the reaction vessel outlets and the drain channels (40) while a relative movement station by station between the plate (16) and the stations (28, 30, 32, 34) takes place.

12. Device according to claim 10 or 11,
**characterized in that** the plate (16) is a rotary plate and **in that** all the liquid feed devices (36) are accommodated in or on a valve-carrying panel (12) and all the drain channels (40) are accommodated in a suction panel (14).

13. Device according to one of claims 10 to 12,
**characterized in that** each liquid feed device (36) has n feed valves (38).

14. Device according to claim 12 or 13,
**characterized in that** the valve-carrying panel (12) and the suction panel (14) are fixed against rotation, and **in that** the rotary plate (16) and the suction panel (14) can be lowered relative to the valve-carrying panel (12).

15. Device according to one of claims 10 to 14,
**characterized in that** each insert (18) extends radially in the plate (16) and has at least one row, preferably two rows parallel to one another, of reaction vessels (20).

16. Device according to one of claims 10 to 15,
**characterized in that** the inserts (18) are made of plastic, in particular of polyether ether ketone.

17. Device according to one of claims 10 to 16,
**characterized in that** each insert (18) has on its longitudinal sides a coding groove (90) of different design or a coding projection (92) cooperating with a corresponding coding projection (90') or a corresponding coding groove (92') of the rotary plate (16).

18. Device according to claim 17,
**characterized in that** the inserts (18) can be assembled to reaction vessel panels (94) by means of the coding grooves (90) and coding projections (92).

19. Device according to one of claims 10 to 18,
**characterized in that** the reaction vessel outlet (26) and/or the reaction vessel inlet (24) of each reaction vessel (20) has a constriction point (96) which can be closed by a movable ball (98).

20. Device according to claim 19,
**characterized in that** the ball (98) is pushed in the direction of the constriction point (96) by the force of gravity, by the force of a spring or by magnetic force.

21. Device according to one of claims 10 to 12,
**characterized in that** at least the liquid feed device (36) of the third station (32) includes
- a plurality of individual feeds (100), each of which is in liquid connection with only one of the various liquids to be added to the reaction vessels (20) at the station and the number of which corresponds to at least the number of different liquids to be added to the reaction vessels (20) at the station,
- a coupling drive (102) for the individual feeds (100), in order to connect each individual feed (100) with a reaction vessel inlet (24) as required, and
- a common carrier (104) for the individual feeds (100) which can be displaced at right angles to the reaction vessel inlets (24).

22. Device according to claim 21,
**characterized in that** the carrier (104) is constructed as a carriage which can be displaced position by position, each reaction vessel inlet (24) representing a position.

23. Device according to claim 21 or 22,
**characterized in that** the coupling drive (102) in a position of the carrier (104) in which individual feeds (100) are in alignment with reaction vessel inlets (24) connects to a reaction vessel inlet (24) only that individual feed (100) which is connected to the liquid precisely required in the reaction vessel (20) in question, and **in that**, depending on the requirement, the carrier (104) is then displaced optionally several times into another position in which individual feeds (100) are in alignment with reaction vessel inlets (24) and in which in turn the coupling drive (102) connects to a reaction vessel inlet (24) only that individual feed (100) which is connected to precisely the liquid required in the reaction vessel (20) in question.

## Revendications

1. Procédé pour la production en parallèle d'au moins 4n oligonucléotides, comprenant les étapes de :
- agencement d'au moins quatre plaques insérables comportant chacune n récipients de réaction sur un plateau de sorte qu'une première plaque insérable se trouve à un premier poste, une deuxième plaque insérable à un deuxième poste, une troisième plaque insérable à un troisième poste et une quatrième plaque insérable à un quatrième poste, chaque récipient de réaction contenant une base de départ liée à un support inerte ou un support universel,
- exécution en parallèle de
a) une opération de déblocage servant à l'élimination des groupes protecteurs et effectuée simultanément dans tous les n récipients de réaction de la plaque insérable se trouvant au premier poste,
b) une première opération de lavage effectuée simultanément dans tous les n récipients de réaction de la plaque insérable se trouvant au deuxième poste,
c) une opération de couplage servant à l'addition des nucléotides individuels et effectuée dans tous les n récipients de réaction de la plaque insérable se trouvant au troisième poste et
d) une deuxième opération de lavage suivie d'une opération d'ajout de coiffe (capping) pour le blocage des oligonucléotides qui n'ont pas trouvé l'élongation souhaitée lors de l'opération de couplage précédente, suivie d'une troisième opération de lavage qui est elle-même suivie d'une opération d'oxydation pour la stabilisation du squelette de phosphate des oligonucléotides, suivie d'une quatrième opération de lavage effectuée simultanément dans tous les n récipients de réaction de la plaque insérable se trouvant au quatrième poste, et
- rotation d'un poste à un autre des plaques insérables passant ainsi par les quatre postes susmentionnés ou rotation des postes par rapport aux plaques insérables et exécution desdites opérations jusqu'à ce que les oligonucléotides désirés soient formés par couplage de nucléotides individuels.

2. Procédé selon la revendication 1,
**caractérisé en ce qu'**il est effectué au deuxième poste, en même temps que la première opération de lavage, une opération de monitorage laquelle renseigne sur la qualité de l'opération de déblocage exécutée au premier poste.

3. Procédé selon la revendication 2,
**caractérisé en ce que** l'opération de monitorage comprend une mesure en ligne de la conductibilité d'un liquide de lavage utilisé pour la première opération de lavage.

4. Procédé selon la revendication 2,
**caractérisé en ce que** l'opération de monitorage comprend une mesure en ligne, notamment une mesure à l'aide de lumière ultraviolette, de l'intensité de coloration d'un liquide de lavage utilisé pour la première opération de lavage.

5. Procédé selon l'une des revendications 2 à 4,
**caractérisé en ce que** la première opération de lavage est exécutée jusqu'à ce que l'opération de monitorage fasse ressortir que les groupes protecteurs retirés lors de l'opération de déblocage précédente ont bien été complètement éliminés.

6. Procédé selon l'une des revendications sus-citées,
**caractérisé en ce qu'**une base nucléotidique sélectionnée est, en même temps qu'un activateur qui sera de préférence du tétrazole, introduite dans les récipients de réaction lors de l'opération de couplage effectuée au troisième poste.

7. Procédé selon l'une des revendications sus-citées,
**caractérisé en ce qu'**un groupe de marquage, en particulier un analogue de base, un colorant ou un haptène, est introduit dans les récipients de réaction lors de l'opération de couplage effectuée au troisième poste.

8. Procédé selon l'une des revendications sus-citées,
**caractérisé en ce que** les récipients de réaction sont conçus pour former des récipients permettant le passage d'un liquide et que les liquides à introduire aux quatre postes dans les récipients de réaction sont introduits dans les récipients de réaction et évacués de ces derniers par différence de pression entre l'ouverture d'entrée et l'ouverture de sortie des récipients de réaction.

9. Procédé selon l'une des revendications sus-citées,
**caractérisé en ce que** toutes les opérations sont effectuées sous atmosphère protectrice, en particulier sous azote et/ou argon.

10. Dispositif pour la production en parallèle d'au moins 4 n oligonucléotides comprenant
- un premier poste (28) permettant l'exécution d'une opération de déblocage,
- un deuxième poste (30) permettant l'exécution d'une première opération de lavage,
- un troisième poste (32) permettant l'exécution d'une opération de couplage,
- un quatrième poste (34) permettant l'exécution d'une seconde opération de lavage suivie d'une opération d'ajout de coiffe (capping) suivie d'une troisième opération de lavage suivie d'une opération d'oxydation suivie d'une quatrième opération de lavage, le premier, le second, le troisième et le quatrième poste se succédant à distance suivant la direction circonférentielle,
- un plateau (16) présentant au moins quatre plaques insérables (18) comportant chacune n récipients de réaction (20) de sorte qu'une première plaque insérable (18) se trouve au premier poste (28), une seconde plaque insérable (18) au second poste (30), une troisième plaque insérable (18) au troisième poste (32) et une quatrième plaque insérable (18) au quatrième poste (34), chaque récipient de réaction (20) étant conçu pour former un récipient permettant le passage d'un liquide et comportant une ouverture d'entrée ménagée dans la partie inférieure et une ouverture de sortie ménagée dans la partie supérieure dudit récipient,
- un dispositif permettant un déplacement d'un poste à un autre par mouvement relatif entre le plateau (16) et les postes (28, 30, 32, 34),
- un dispositif d'amenée de liquide (36) associé, dans chaque poste (28, 30, 32, 34), directement aux ouvertures d'entrée des récipients de réaction, et
- un conduit d'évacuation (40) associé, dans chaque poste (28, 30, 32, 34), directement à chaque ouverture de sortie des récipients de réaction, chaque ouverture d'entrée des récipients de réaction étant en prise de manière étanche avec le dispositif d'amenée de liquide (36) associé et chaque ouverture de sortie desdits récipients étant en prise de manière étanche avec le conduit d'évacuation (40) associé lorsque les plaques insérables (18) se trouvent à un poste (28, 30, 32, 34), et une faible distance axiale existant pour le moins entre les ouvertures d'entrée des récipients de réaction et le dispositif d'amenée de liquide (36) lorsqu'est effectué, par poste, un mouvement relatif entre le plateau (16) et les postes (28, 30, 32, 34).

11. Dispositif selon la revendication 10,
**caractérisé en ce qu'**il existe une faible distance axiale également entre les ouvertures de sortie des récipients de réaction et les conduits d'évacuation (40) lorsqu'est effectué, par poste, un mouvement relatif entre le plateau (16) et les postes (28, 30, 32, 34).

12. Dispositif selon la revendication 10 ou 11,
**caractérisé en ce que** que le plateau (16) est un plateau rotatif et que tous les dispositifs d'amenée de liquide (36) sont logés dans ou sur une plaque support de valves (12) et tous les conduits d'évacuation (40) sont logés dans une plaque d'aspiration (14).

13. Dispositif selon l'une des revendications 10 à 12,
**caractérisé en ce que** chaque dispositif d'amenée de liquide (36) présente n valves d'amenée (38).

14. Dispositif selon la revendication 12 ou 13,
**caractérisé en ce que** la plaque support de valves (12) et la plaque d'aspiration (14) sont fixes en rotation et que le plateau rotatif (16) et la plaque d'aspiration (14) sont abaissables par rapport à la plaque support de valves (12).

15. Dispositif selon l'une des revendications 10 à 14,
**caractérisé en ce que** chaque plaque insérable (18) s'étend suivant la direction radiale dans le plateau (16) et présente au moins une rangée, de préférence deux rangées parallèles, de récipients de réaction (20).

16. Dispositif selon l'une des revendications 10 à 15,
**caractérisé en ce que** les plaques insérables (18) sont constituées d'un matériau synthétique, notamment de polyétheréther-cétone.

17. Dispositif selon l'une des revendications 10 à 16,
**caractérisé en ce que** chaque plaque insérable (18) présente sur ses grands côtés une rainure de codage (90) de conception différente ou une saillie de codage (92) qui coopère avec une saillie de codage (90') ou une rainure de codage (92') correspondante du plateau (16).

18. Dispositif selon la revendication 17
**caractérisé en ce que** les plaques insérables (18) peuvent être assemblées en plaques de récipients de réaction à l'aide des rainures de codage (90) et des saillies de codage (92).

19. Dispositif selon l'une des revendicatins 10 à 18,
**caractérisé en ce que** l'ouverture de sortie (26) et/ou l'ouverture d'entrée (24) de chaque récipient de réaction (20) présente un goulot d'étranglement (96) pouvant être obturé par une bille mobile (98).

20. Dispositif selon la revendication 19,
**caractérisé en ce que** la bille (98) est poussée par force de pesanteur, par force de ressort ou par force magnétique vers le goulot d'étranglement (96).

21. Dispositif selon l'une des revendication 10 à 12,
**caractérisé en ce que** pour le moins le dispositif d'amenée de liquide (36) du troisième poste (32) présente
- plusieurs conduits d'amenée individuels (100) dont chacun d'eux est en liaison liquide avec seulement un des différents liquides à introduire au poste dans les récipients de réaction (20) et dont le nombre correspond au moins au nombre des différents liquides à introduire au poste dans les récipients de réaction (20),
- un actionneur de couplage (102) pour les conduits d'amenée individuels (100) pour relier sélectivement chaque conduit d'amenée individuel (100) à une ouverture d'entrée des récipients de réaction, et
- un support (104) commun pour les conduits d'amenée individuels (100) qui peut se déplacer perpendiculairement aux ouvertures d'entrée (24) des récipients de réaction.

22. Dispositif selon la revendication 21,
**caractérisé en ce que** le support (104) est conçu pour former un chariot pouvant être déplacé d'une position à une autre, chaque ouverture d'entrée (24) des récipients de réaction représentant une position.

23. Dispositif selon la revendication 21 ou 22,
**caractérisé en ce que**, dans une position du support (104) dans laquelle les conduits d'amenée individuels (100) sont alignés sur les ouvertures d'entrée (24) des récipients de réaction, l'actionneur de couplage (102) relie à une ouverture d'entrée (24) des récipients de réaction uniquement le conduit d'amenée individuel (100) qui est en contact avec le liquide justement nécessité par le récipient de réaction (20) concerné, et que le support (104) est, en fonction des besoins, ensuite amené éventuellement à plusieurs reprises dans une autre position dans laquelle des conduits d'amenée individuels (100) sont alignés sur des ouvertures d'entrée (24) des récipients de réaction et dans laquelle l'actionneur de couplage (102) relie à une ouverture d'entrée (24) des récipients de réaction uniquement le conduit d'amenée individuel (100) qui est en contact avec le liquide justement nécessité par le récipient de réaction concerné.
